(19) Europäisches Patentamt
European Patent Office
Office européen des brevets

(11) Veröffentlichungsnummer : **0 026 281**
**B1**

(12) # EUROPÄISCHE PATENTSCHRIFT

(45) Veröffentlichungstag der Patentschrift : **15.12.82**

(51) Int. Cl.³ : **C 07 C 103/78**, A 61 K 49/04

(21) Anmeldenummer : **80104243.3**

(22) Anmeldetag : **18.07.80**

(54) **Neue Derivate der 2,4,6-Trijod-isophthalsäure, Verfahren zu deren Herstellung und diese enthaltende Röntgenkontrastmittel.**

(30) Priorität : **09.08.79 IT 2502679**

(43) Veröffentlichungstag der Anmeldung : **08.04.81 (Patentblatt 81/14)**

(45) Bekanntmachung des Hinweises auf die Patenterteilung : **15.12.82 Patentblatt 82/50**

(84) Benannte Vertragsstaaten : **AT BE CH DE FR GB LI NL SE**

(56) Entgegenhaltungen :
**EP A 0 015 867**
**CH A 544 551**
**CH A 616 403**
**DE A 2 547 789**

**Die Akte enthält technische Angaben, die nach dem Eingang der Anmeldung eingereicht wurden und die nicht in dieser Patentschrift enthalten sind.**

(73) Patentinhaber : **BRACCO INDUSTRIA CHIMICA Società per Azioni**
**Via E. Folli, 50**
**I-20134 Milano (IT)**

(72) Erfinder : **Felder, Ernst, Prof. Dr.**
**Via Ceresio 49**
**CH-6826 Riva S. Vitale (CH)**
Erfinder : **Pitrè, Davide, Prof. Dr.**
**Via Brocchi 24**
**Milano (IT)**

(74) Vertreter : **Zutter, Hans Johann Niklaus**
**EPROVA Aktiengesellschaft im Laternenacker 5**
**CH-8200 Schaffhausen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 026 281 B1

**0 026 281**

Neue Derivate der 2,4,6-Trijod-isophthalsäure, Verfahren zu deren Herstellung und diese enthaltende Rontgenkontrastmittel

Die vorliegende Erfindung betrifft die neuen in Wasser leicht löslichen 5-(N-Alkyl-α-hydroxyacyl-amino)-2,4,6-trijod-isophthalsäure-bis-(hydroxyalkyl-amide) der allgemeinen Formel (I)

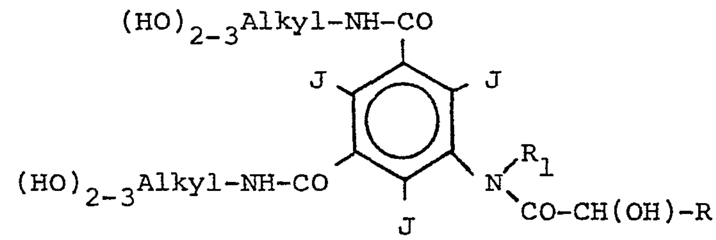

(I)

worin

$(HO)_{2-3}$Alkyl-1,3-Dihydroxyisopropyl-, 2,3-Dihydroxypropyl- oder 1,3-Dihydroxy-2-hydroxymethyliso-propyl-,

R Wasserstoff oder Methyl-,

$R_1$ einen Alkyl-Rest mit 1 bis 5 C-Atomen

bedeutet, das Verfahren zu ihrer Herstellung sowie nicht ionische Röntgenkontrastmittel, die insbesondere zur Vasographie, Urographie und zur Kontrastverstärkung in der Computertomographie geeignet sind, und die mindestens eine der vorstehend genannten Verbindungen als schattengebende Komponente enthalten. Bevorzugte Alkyl-Reste $R_1$ sind Methyl, Äthyl, Propyl.

5-Acylamino-2,4,6-trijod-isophthalsäure-diamide und deren Anwendung in Röntgenkontrastmitteln sind aus der CH-PS 544-551 bekannt. Sie enthalten nur einfache, unsubstituierte aliphatische Acyl-Gruppen in der Regel Acetyl-Gruppen. Einige Kohlenhydrat-Reste aufweisende Vertreter dieser Gruppe sind ausreichend wasserlöslich, beispielsweise das unter dem Freinamen METRIZAMIDE bekannt gewordene 3-Acetylamino-5-N-methyl-acetylamino-2,4,6-trijod-benzoylglucosamin. Vergl. dazu Verbindung Nr. 11 der US-PS 3.701.771, GB-PS 1.321.591, CH-PS 554.551, AT-PS 318.134 bzw. der DE-OS 2.031.724 ; Publikationen von T. Almen, S. Salvesen, K. Golman ; Acta Radiologia Suppl. 335 (1973), 1-13, 233-75, 312-38. Nachteilig ist seine schwierige Zugänglichkeit, das Vorliegen in Form eines praktisch untrennbaren Isomerengemisches und namentlich seine relativ geringe Stabilität in wässriger Lösung, welche die Verwendbarkeit wesentlich einschränkt und die Handhabung erschwert.

Einen Fortschritt stellte das unter dem Freinamen IOPAMIDOL bekannt gewordene L-5-α-Hydroxy-propionylamino-2,4,6-trijod-isophthalsäure-bis-(1,3-dihydroxyisopropylamid) dar. Vergleiche dazu DE-PS 2.547.789, GB-PS 1.472.050, US-PS 4.001.323, Felder et al., Il FARMACO, Ed. Sc. *32* 835-844 (1977). Es zeichnet sich aus durch eine wesentlich einfachere Struktur, durch höhere Stabilität, leichte Isolierbarkeit und durch eine geringere Viskosität seiner konzentrierten wässrigen Lösungen. Die Toxizität dieser Verbindung ist sehr niedrig.

Vor kurzem sind aus der BE-PS 855.580 zwei weitere Derivate der 5-Acylamino-2,4,6-trijod-isophthalsäure bekannt geworden, nämlich 5-(N-2-Hydroxyäthyl-acetyl-amino)-2,4,6-trijod-isophthalsäure-bis-(2,3-dihydroxypropylamid) und 5-(N-2,3-Dihydroxypropyl-acetylamino)-2,4,6-trijod-isophthalsäure-bis-(2,3-dihydroxypropylamid), die ähnliche Eigenschaften aufweisen. Sie leiten sich ab von dem in Wasser kaum löslichen 5-Acetylamino-2,4,6-trijod-isophthalsäure-bis-(2,3-dihydroxy propylamid) ; Wasserlöslichkeit 1 % (g/v) bei 20-40 °C. Es kann daher nicht verwundern, dass einige einschlägige Isomere in Wasser unlöslich und daher praktisch unbrauchbar sind. Vergl. BE-PS 855.580, Seite 21 bis 22.

Die Entwicklung in den letzten Jahren hat deutlich gezeigt, dass es äusserst schwierig ist und nur selten gelingt Verbindungen zu finden, welche die Eigenschaften aufweisen, die heute für breit anwendbare nicht ionische Röntgenkontrastmittel erforderlich sind. Diese Eigenschaften sind echte Wasserlöslichkeit ausreichend zur Herstellung stabiler, d.h. nicht übersättigter konzentrierter Lösungen, maximale allgemeine und neurotrope Verträglichkeit, minimale Osmolalität, geringe Viskosität, maximale Stabilität gegen hydrolytische. Einflüsse und ausreichend einfache Struktur zur Ermöglichung einer oekonomischen Synthese sowie zur Erleichterung der Isolierung und Reinigung.

Zu dieser auserlesenen Gruppe von schattengebenden Komponenten gehören auch die vorliegenden neuen Verbindungen der Formel (I).

Sie zeichnen sich gesamthaft aus durch hohe Wasserlöslichkeit, die bei einzelnen Vertretern absolute Spitzenwerte erreicht, durch optimale Verträglichkeit und relativ geringe Osmolalität sowie mit ihrer hohen Stabilität gegenüber hydrolytischen Einflüssen, wobei sie die an sich schon gute Stabilität der ihnen zugrundeliegenden am aromatischen N-Atom nicht alkylierten Ausgangsstoffe noch deutlich übertreffen. Diese verstärkte Stabilität gegen hydrolytische Einflüsse ist wichtig zur Verhinderung von auch nur spurenweiser Bildung freier aromatischer Amine im Hinblick auf mögliche aber unzulässige cytotoxische Effekte derselben in Verbindung mit Röntgenstrahlen. Vergleiche dazu : A. Norman et al, Radiology *129*. 199-203 (Okt. 1978).

Die Viskosität der wässrigen Lösungen dieser Verbindungen ist stark abhändig von ihrer spezifischen

Struktur. Sie kann im Rahmen der beanspruchten Verbindungen sehr stark variieren und damit den unterschiedlichen Anforderungen des jeweiligen Anwendungszweckes optimal angepasst werden.

Besonders überraschend und zugleich wertvoll ist, dass durch die Einführung auch von niedrigen unsubstituierten, also hydrophoben, Alkyl-Resten an das aromatische N-Atom in 5-Stellung der zugrundeliegenden Verbindung — zum Beispiel das L-5-α-Hydroxypropionylamino-2,4,6-trijod-isophthalsäure-bis-(1,3-dihydroxyisopropylamid) = IOPAMIDOL oder das 5-Hydroxyacetylamino-2,4,6-trijod-isophthalsäure-bis-(2,3-dihydroxypropylamid) — die Wasserlöslichkeit nicht nur erhalten bleibt sondern besonders bei den N-Methyl-Verbindungen sogar beträchtlich verstärkt wird und ausserdem auch die Stabilität gegenüber hydrolytischen Einflüssen erhöht wird.

IOPAMIDOL hat bei 20 °C eine Wasserlöslichkeit entsprechend 440 mg J/ml [d.s. 89,7 % (g/v)] das Hydrat entsprechend 307 mg J/ml [d.s. 62,7 % (g/v)]. Die 5-N-Alkyl-Derivate, welche unter die vorliegende Erfindung fallen, weisen dagegen Wasserlöslichkeiten von 100 % (g/v) auf. Zudem ist ihre Hydrolysestabilität höher als die der entsprechenden nicht-alkylierten Verbindungen.

Die erfindungsgemässen neuen Röntgenkontrastmittel haben aufgrund ihrer hervorragenden Eigenschaften, insbesondere ihrer guten Wasserlöslichkeit, ihres nicht-ionogenen Charakters, ihrer hohen Stabilität, sehr guten Verträglichkeit und vergleichsweise relativ einfachen Struktur ein sehr breites Anwendungsspektrum. Sie lassen sich recht oekonomisch herstellen und daher auch für Zwecke verwenden, bei denen die zulässigen Kosten für das Kontrastmittel beschränkt sind.

Schwerpunkte ihrer Anwendung sind die Gefässdarstellungen also die Angiographien, wie z.B. die Artheriographie, die Darstellung des Herzens (Cardiographie) und der Herzkranzgefässe (Coronarographie), die abdominale, die selektiv abdominale und die thorakale Aortographie, ferner die renale und cerebrale Angiographie, die Phlebographie sowie die Urographie und die Kontrastverstärkung in der Computertomographie. Bei der letztgenannten Anwendung werden sehr grosse Mengen von Kontrastmittel benötigt, beispielsweise 250 ml kontrastmittellösung mit 300 mg Jod/ml, enthaltend insgesamt 75 g Jod. Verständlicherweise sind bei derartig grossen Gaben für rein diagnostische Zwecke die Anforderungen an die Verträglichkeit und Sicherheit ungewöhnlich hoch. Weitere Anwendungsgebiete sind beispielsweise die Bronchographie, die Darstellung von Körperhöhlen und der Liquorräume sowie die Lymphangiographie.

Die 5-(N-Methyl-α-hydroxyacyl-amino)-2,4,6-trijod-isophthalsäure-bis-(hydroxyalkylamide) der allgemeinen Formel (II)

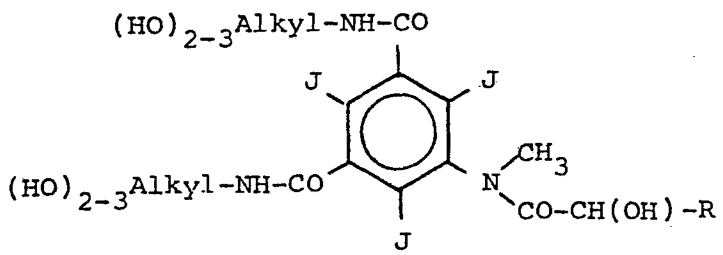

(II)

worin

$(HO)_{2-3}$Alkyl 1,3-Dihydroxyisopropyl-, 2,3-Dihydroxypropyl- oder 1,3-Dihydroxy-2-hydroxymethylisopropyl- und

R Wasserstoff oder Methyl

bedeutet, sind schattengebende Komponenten, die sich im allgemeinen durch besonders hohe Wasserlöslichkeit und niedrige Viskosität auszeichnen. Sie sind für die vorgenannten Anwendungen hervorragend geeignet.

Das Verfahren zur Herstellung der als schattengebende Komponenten in Röntgenkontrastmitteln verwendbaren 5-(N-Alkyl-α-hydroxyacyl-amino)-2,4,6-trijod-isophthalsäure-bis-(hydroxyalkylamide) der Formel I ist dadurch gekennzeichnet, dass man ein 5-α-Hydroxyacylamino-2,4,6-trijod-isophthalsäure-bis-(dihydroxypropylamid) der allgemeinen Formel (III)

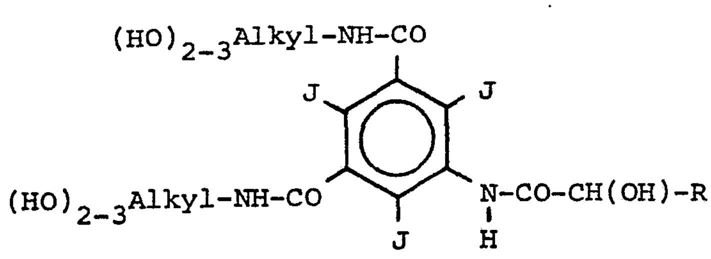

(III)

am aromatischen Stickstoff-Atom in alkalischen Milieu alkyliert durch Umsetzung mit Alkylierungsmitteln der allgemeinen Formel (IV)

$R_1-X$ (IV)

wobei R und $R_1$ in den Formeln (III) bzw. (IV) die auf Seite 1 definierte Bedeutung hat und X eines der Halogen-Atome Jod, Brom oder Chlor oder ein Sulfat-bzw. Sulfonat-Radikal ($-OSO_2-OR_1$ bzw. $-OSO_2$-Alkyl oder $-OSO_2$-Aryl) bedeutet, oder dass man ein reaktives funktionelles Derivat einer 5-(N-Alkyl-α-hydroxyacyl-amino)-2,4,6-trijod-isophthalsäure der allgemeinen Formel (V)

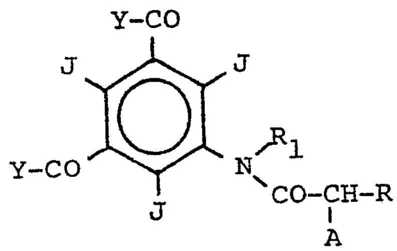

(V)

worin R und $R_1$ die weiter oben definierte Bedeutung haben, A einen niedrigen Acyloxy-Rest mit 1 bis 5 C-Atomen oder ein Halogen-Atom und Y—CO— reaktive Säurehalogenid oder -anhydrid-Radikale bedeuten, mit einem Di- oder Trihydroxyalkylamin $(HO)_{2-3}Alkyl-NH_2$, dessen Hydroxy-Funktionen durch Acetalisierung oder Ketalisierung maskiert sein können, umsetzt und im erhaltenen 5-(N-Alkyl-α-hydroxyacyl-amino)-2,4,6-trijod-isophthalsäure-bis-(hydroxyalkylamid)-Derivat die maskierende Funktion A und gegebenenfalls vorhandene Acetal- oder Ketal-Funktionen hydrolytisch in die Hydroxy-Funktionen spaltet.

Ein entsprechendes 5-α-Hydroxyacylamino-2,4,6-trijod-isophthalsäure-bis-(hydroxyalkylamid) wird demnach in Gegenwart von Basen mit einem Alkylhalogenid, Alkylsulfat oder einem entsprechenden Sulfonsäure-alkylester z.B. Methan-, Benzol- oder Toluolsulfonsäure-alkylester umgesetzt.

Typische konkrete Beispiele für Alkylierungsmittel der Formel $R_1$-X sind :

Methylbromid, Methyljodid, Methylchlorid, Dimethylsulfat, Methansulfonsäure-methylester, Benzolsulfonsäure-methylester, Toluolsulfonsäure-methylester, Äthylbromid, Äthyljodid, Äthylchlorid, Diäthylsulfat, Methansulfonsäure-äthylester, Benzolsulfonsäure-äthylester, Toluolsulfonsäure-äthylester, Propylbromid, Propyljodid, Propylsulfat, Methansulfonsäure-propylester, Benzolsulfonsäure-propylester, Toluolsulfonsäure-propylester, Butylbromid, Butyljodid, Dibutylsulfat, Methansulfonsäure-butylester, Benzolsulfonsäure-butylester, Toluolsulfonsäure-butylester, Amyljodid, Amylbromid, Methansulfonsäure-amylester, Benzolsulfonsäure-amylester, Toluolsulfonsäure-amylester.

Die bei der Alkylierung frei werdende starke Säure (HX) wird durch die anwesende Base abgefangen. Als Basen kommen beispielsweise in Betracht : starke Alkalien wie z.B. Alkalialkoholate (NaOME, NaOEt, KOMe, KOEt, LiOMe, LiOEt), Alkalihydroxyde (NaOH, KOH, LiOH), Alkalicarbonate ($Na_2CO_3$, $K_2CO_3$), quarternäre Ammoniumhydroxyde (Tetramethylammoniumhydroxyd). Die Umsetzung wird gewöhnlich in einem polaren Lösungsmittel wie z.B. in Wasser, niedrigen Alkoholen (MeOH, EtOH, Aethylenglykol, Propylengykol, Glycerin) niedrigen Glykoläthern (Methoxyäthanol) Äthoxyäthanol, Butyloxyäthanol), Ketonen (Aceton, Methyläthylketon, Methylisopropylketon, Methylisobutylketon) oder in ausgesprochen aprotischen Lösungsmitteln wie z.B. Hexametanol (MPT), Dimethylformamid (DMF), Dimethylacetamid (DMAC), Dimethylsulfoxyd (DMSO), oder in Lösungsmittelgemischen durchgeführt. Durch Erwärmen wird die Umsetzung beschleunigt.

Reaktionsschema (vereinfacht) :

(Siehe das Schema, Seite 5)

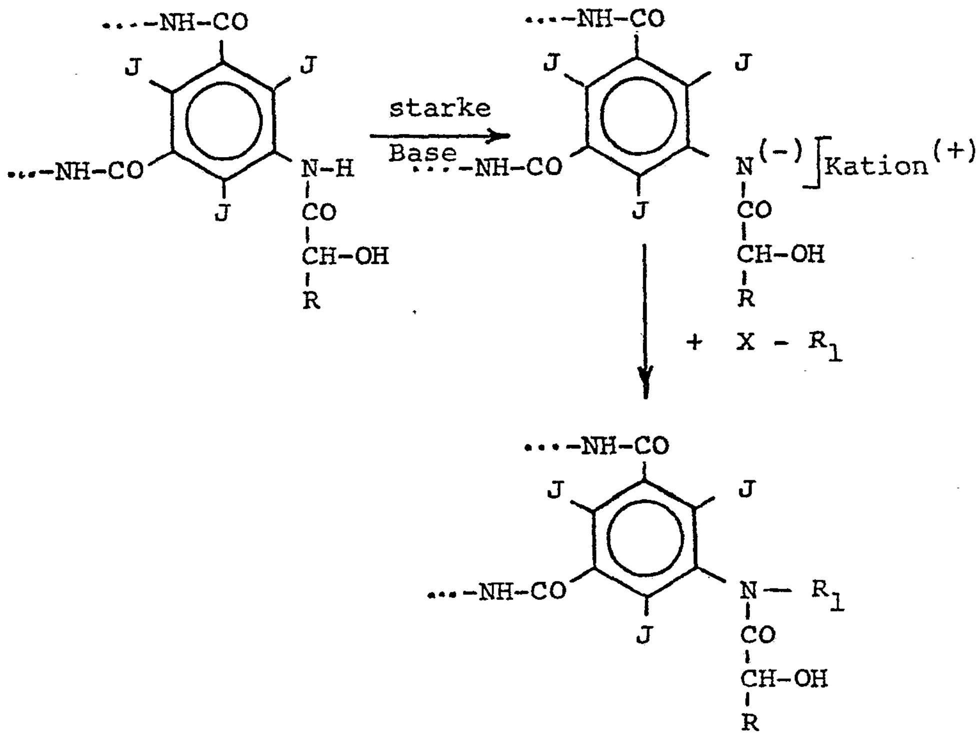


Man kann aber auch ein reaktives Derivat einer 5-(N-Alkyl-α-hydroxyacyl-amino(-2,4,6-trijod-isophthalsäure der allgemeinen Formel (V) bereiten und diese mit einem Dihydroxypropylamin, oder einem funktionellen Derivat davon, umsetzen und im erhaltenen Produkt die maskierenden Gruppen hydrolytisch abspalten und alle Hydroxy-Funktionen in Freiheit setzen.

Als reaktive Säure-Derivate kommen für diese Umsetzung vorzugsweise in Betracht : deren Säurehalogenide insbesondere deren Säurechloride d.h. ein 5-(N-Alkyl-α-acyloxyacyl-amino)-2,4,6-trijod-isophthalsäure-dichlorid oder ein entsprechendes Säure-anhydrid mit einer organischen oder anorganischen Säure. Als geeignete organische Säuren sind beispielsweise zu nennen : niedrige Fettsäuren wie z.B. Propionsäure, Buttersäure, Valeriansäure oder Kohlensäure-halbester wie z.B Kohlensäure-mono-methyl-, Kohlensäure-mono-äthyl- oder Kohlensäure-mono-benzylester. Als geeignete anorganische Säuren kommen in Betracht : Stickstoffwassersäure, Schwefelsäure-halbester, Phosphorsäure, phosphorige Säure, Phosphorigsäure-dialkylester, z.B. Phosphorigsäure-diäthylester.

Die Umsetzung mit einem Dihydroxypropylamin oder einem Derivat davon wird gewöhnlich in einem hinsichtlich dieser Reaktion inerten Lösungsmittel beispielsweise in einem aprotischen Lösungsmittel wie DMF, DMAC usw. innerhalb eines Temperaturbereiches von ca. – 10 °C bis ca. + 150 °C durchgeführt.

Als Hydroxyalkylamine oder deren Derivate werden vorzugsweise folgende Verbindungen zur Umsetzung benutzt : 1,3-Dihydroxyisopropylamin (Serinol), 2,3-Dihydroxypropylamin, Tris-(hydroxymethyl)-aminomethan [2-Amino-2-hydroxymethyl-1,3-propandiol] sowie Ketale oder Acetale davon, beispielsweise 5-Amino-2,2-dimethyl-1,3-dioxan, 4-Aminomethyl-2,2-dimethyl-1,3-dioxolan, 5-Amino-2-methyl-1,3-dioxan, 5-Amino-2-phenyl-1,3-dioxan oder 5-Amino-1,3-dioxan.

Für die Einführung der Hydroxyacyl-Reste und einige Umsetzungen der diese enthaltenden Verbindungen muss die Hydroxy-Funktion maskiert werden. Gewöhnlich benutzt man dazu eine Acyloxy-Funktion A bestehend aus einem niedrigen Acyloxy-Rest, vorzugsweise dem Acetyloxy-Rest, die sich bei der Schlussstufe leicht durch alkalische Verseifung in die Hydroxy-Funktion umwandeln lässt.

Besonders für die Herstellung der Hydroxyacetyl-Derivate kann man auch von entsprechenden leicht zugänglichen Halogenacetyl-Verbindungen der Formel (V), worin R = H und A = Halogen, vorzugsweise Chlor, bedeutet, ausgehen. Durch alkalische Verseifung wird die Halogenacetyl-Gruppe auf sehr einfache Weise in die schlussendlich gewünschte Hydroxyacetyl-Gruppe verwandelt.

## Beispiele

### Beispiel 1

L-5-(N-Methyl-α-hydroxypropionyl-amino)-2,4,6-trijodisophthalsäure-bis-(1,3-dihydroxyisopropylamid)

58,3 g L-5-α-Hydroxypropionyl-amino-2,4,6-trijod-isophthalsäure-bis-(1,3-dihydroxyisopropylamid) (0,075 Mol) werden in 200 ml Wasser gelöst und mit genau der stöchiometrischen Menge (0,075 Mol) 2N NaOH

versetzt. Die Lösung hat ein pH von 11,9. Sie wird im Vakuum zur Trockene eingedampft. Der Rückstand bestehend aus der 5 N-Natrium-Verbindung (Na-Salz) von L-5-α-Hydroxypropionyl-amino-2,4,6-trijod-isophthalsäure-bis-(1,3-dihydroxyisopropylamid) wird im Vakuum bei 100 °C getrocknet.

Aequivalentgewicht für $C_{17}H_{21}J_3N_3NaO_8$ ber. 799,27 gef. 799,08.

Das so erhaltene Na-Salz (60 g = 0,075 Mol) wird in 220 ml Dimethylacetamid (DMAC) gelöst und bei 30 °C tropfenweise mit 12,7 g Methyljodid (0,09 Mol) versetzt. Man rührt ca. 1 h bei 40 °C bis die Umsetzung aufgrund chromatographischer Kontrollen praktisch vollständig ist. Die Reaktionslösung wird im Vakuum eingedampft. Der sirupöse Rückstand wird in 600 ml Aceton eingerührt, wobei das Produkt (und NaJ) ausfällt. Die Fällung wird abfiltriert, in 400 ml Wasser gelöst und durch Perkolation durch eine mit Kationenaustauscherharz (z.B. Amberlite(R) IR 120) beschickte Säule und anschliessend durch Perkolation durch eine mit Anionenaustauscherharz (z.B. Amberlite(R) IR 45) beschickte Säule vollständig entsalzt.

Das Säuleneluat wird vollständig zur Trockene eingedampft. Ausbeute : 42,2 g L-5-(N-Methyl-α-hydroxypropionyl-amino)-2,4,6-trijod-isophthalsäure-bis-(1,3-dihydroxyisopropylamid), das sind 71 % der Theorie.

Schmelzpunkt (nach Umkristallisieren aus abs. Äthanol) ca. 250 °C (sintern bei 190 °C).

Dünnschichtchromatogramm (DC) auf Kieselgel : Laufmittel Chloroform/Methanol/Ammoniak (25 %) = 6 : 3 : 1.

Rf = 0,29 und 0,33.

$C_{18}H_{24}J_3N_3O_8$ : J ber. 48,12 % gef. 47,99 %.

Wasserlöslichkeit : ≥ 100 % (g/v) bei 25 °C.

Dieselbe Verbindung wird auch erhalten, wenn man beim vorstehend beschriebenen Ansatz das Methyljodid durch 11,4 g Dimethylsulfat 0,09 Mol) ersetzt und sonst in gleicher Weise verfährt.

Völlig analog wird durch Methylierung des in Wasser nur beschränkt löslichen D,L-5-α-Hydroxypropionylamino-2,4,6-trijod-isophthalsäure-bis-(1,3-dihydroxyisopropylamides) das D,L-5-(N-Methyl-α-hydroxypropionyl-amino)-2,4,6-trijod-isophthalsäure-bis-(1,3-dihydroxyisopropylamid) erhalten.

Schmelzpunkt : 298-300 °C unter Zersetzung.

DC : Rf = 0,34 und 0,39 mit $CHCl_3/MeOH/NH_4OH$ = 6 : 3 : 1.

Dieses Produkt löst sich in Wasser spielend leicht. Die Lösungen sind jedoch übersättigt.

## Beispiel 2

L-5(N-Äthyl-α-hydroxypropionyl-amino)-2,4,6-trijod-isophthalsäure-bis-(1,3-dihydroxyisopropylamid)

90 g 5N-Natrium-Verbindung von L-5-α-Hydroxypropionylamino-2,4,6-trijod-isophthalsäure-bis-(1,3-dihydroxyisopropylamid (0,112 Mol) werden in 240 ml DMAC mit 26,5 g Äthyljodid (0,17 Mol) umgesetzt und analog Beispiel 1 aufgearbeitet. Man erhält 66 g der Titelverbindung, d.s. 73 % der Theorie.

Schmelzpunkt : 295-297 °C Zersetzung.

DC : RF = 0,27. Laufmittel $CHCl_3/MeOH/NH_4OH$ (25 %ig) = 6 : 3 : 1.

$C_{19}H_{26}J_3N_3O_8$ : J ber. 47,28 % gef. 47,21 %.

$[\alpha]_D^{20} = + 18{,}83°$ (c = 10 % in Wasser).

## Beispiel 3

L-5-(N-Propyl-α-hydroxypropionyl-amino)-2,4,6-trijod-isophthalsäure-bis-(1,3-dihydroxyisopropylamid)

38 g 5 N-Natrium-Verbindung von L-5-α-Hydroxypropionylamino-2,4,6-trijod-isophthalsäure-bis-(1,3-dihydroxyisopropylamid) (0,045 Mol) in 120 ml DMAC werden analog Beispiel 1 mit 7,5 g Propylbromid (0,06 Mol) bei 80 °C umgesetzt. Durch Verteilung zwischen Butanol und Wasser kann das Produkt entsalzt werden.

Ausbeute : 18,43 g L-5-(N-Propyl-α-hydroxypropionyl-amino)-2,4,6-trijod-isophthalsäure-bis-(1,3-dihydroxyisopropylamid), das sind 50 % der Theorie.

Schmelzpunkt : 149 °C (sintern bei 142 °C).

DC : Rf = 0,35 ; 0,42 und 0,48. Laufmittel : $CH_2Cl_2/CHCl_3$ = 10 : 3.

$C_{20}H_{26}J_3N_3O_8$ : J ber. 46,47 % gef. 46,25 %.

Wasserlöslichkeit : ≥ 100 % (g/v) bei 25 °C.

## Beispiel 4

L-5-(N-Butyl-α-hydroxypropionyl-amino)-2,4,6-trijod-isophthalsäure-bis-(1,3-dihydroxyisopropylamid)

80 g Na-Salz von L-5-α-Hydroxypropionylamino-2,4,6-trijodisophthalsäure-bis-(1,3-dihydroxyisopropylamid) = IOPAMIDOL werden in 240 ml DMAC bei 40-80 °C mit 17,8 g Butylbromid (0,13 Mol) umgesetzt.

Durch Verteilung zwischen Methyläthylketon und Wasser (Gegenstromextraktion) lässt sich das Produkt entsalzen.

Ausbeute : 30 g L-5-(N-Butyl-α-hydroxypropionyl-amino)-2,4,6-trijod-isophthalsäure-bis-(1,3-dihydroxyisopropylamid).

Schmelzpunkt (nach Umfällen aus Isopropanol/Diisopropyläther und wiederholtem Umlösen in Wasser) : 140-145 °C.

DC : Rf = 0,36 ; 0,46 und 0,51. Laufmittel $CH_2Cl_2$/MeOH = 10 : 3.

$C_{21}H_{30}J_3N_3O_8$ : J ber. 45,69 % gef. 45,88 %.

Wasserlöslichkeit : ⩾ 100 % (g/v).

Beispiel 5

5-N-Methyl-hydroxyacetyl-amino)-2,4,6-trijod-isophthalsäure-bis-(1,3-dihydroxyisopropylamid)

50 g Na-Salz von 5-Hydroxyacetylamino-2,4,6-trijod-isophthalsäure-bis-(1,3-dihydroxyisopropylamid) (0,064 Mol) in 250 ml DMAC werden mit 13,8 g Methyljolid analog Beispiel 1 umgesetzt.

Man erhält 37,9 g 5-(N-Methyl-hydroxyacetyl-amino)-2,4,6-trijod-isophthalsäure-bis-(1,3-dihydroxyisopropylamid), das sind 77 % der Theorie.

Schmelzpunkt : 215-220 °C.

DC : RF = 0,45. Laufmittel Äthylacetat/Eisessig/Wasser = 15 : 3 : 5.

$C_{17}H_{22}J_3N_3O_8$ : J ber. 48,99 % gef. 48,61 %.

Das als Zwischenprodukt verwendete 5-Hydroxyacetylamino-2,4,6-trijod-isophthalsäure-bis-(1,3-dihydroxyisopropylamid) wird nach in der DE-PS 2 547 789 beschriebenen Methode wie folgt erhalten :

A. 5-Amino-2,4,6-trijod-isophthalsäure-dichlorid (59,6 g) wird in DMAC mit 34 g Acetoxyacetylchlorid (0,25 Mol) umgesetzt, wobei 67,5 g 5-Acetoxyacetylamino-2,4,6-trijod-isophthalsäure-dichlorid vom Schmelzpunkt 234-235 °C erhalten werden.

B. 150 g 5-Acetoxyacetylamino-2,4,6-trijod-isophthalsäure-dichlorid werden in 810 ml DMAC mit 80 g Tributylamin und danach mit 49,2 g Serinol [= 1,3-Dihydroxyisopropylamin] in 540 ml DMAC versetzt. Man erhält 172 g 5-Acetoxyacetylamino-2,4,6-trijod-isophthalsäure-bis-(1,3-dihydroxyisopropylamid), welches etwa bei 190-192 °C unter Zersetzung schmilzt. Diese Verbindung wird in Wasser suspendiert und bei 45 °C vorsichtig mit 1N NaOH bei pH 11 behandelt, bis die Acetoxy-Gruppe vollständig hydrolysiert ist.

Die erhaltene Lösung wird durch Perkolation durch eine mit Kationenaustauscherharz (Amberlite(R) IR 120) und eine mit Anionenaustauscherharz (Amberlite(R) IR 45) beschickte Säule entsalzt. Das Eluat wird zur Trockene eingedampft und in 90 %igem Äthanol aufgenommen, wobei das gewünschte Zwischenprodukt 5-Hydroxyacetylamino-2,4,6-trijod-isophthalsäure-bis-(1,3-dihydroxyisopropylamid) (73 g) kristallin anfällt.

Schmelzpunkt 300 °C unter Zersetzung.

Beispiel 6

5-(N-Äthyl-hydroxyacetyl-amino)-2,4,6-trijod-isophthalsäure-bis-(1,3-dihydroxyisopropylamid)

Erhalten durch Umsetzung von 50 g Na-Salz von 5-Hydroxyacetylamino-2,4,6-trijod-isophthalsäure-bis-(1,3-dihydroxyisopropylamid) mit Äthyljodid.

Schmelzpunkt : 210 °C.

$C_{18}H_{24}J_3N_3O_8$ : J ber. 48,12 % gef. 48,10 %.

Beispiel 7

L-5-(N-Methyl-α-hydroxypropionyl-amino)-2,4,6-trijodisophthalsäure-bis-(1,3-dihydroxyisopropylamid) Alternative Synthese

Eine Lösung von 14,5 g L-5-(N-Methyl-α-acetoxypropionylamino)-2,4,6-trijod-isophthalsäure-dichlorid (0,02 Mol) in 35 ml DMF wird unter Rühren bei 0-2 °C tropfenweise mit 9,1 g Serinol [= 1,3-Dihydroxyisopropylamin] (0,1 Mol) in 30 ml DMF versetzt. Man rührt noch 3 Stunden bei 20 °C und dampft anschliessend die Reaktionslösung bis zum Sirup ein. Der Rückstand kristallisiert beim Verreiben mit Dichlormethan. Das rohe Produkt wird in 100 ml Wasser aufgenommen, durch Evakuieren von anhaftendem Lösungsmittel befreit und bei 40-50 °C mit wässriger 2N Natriumhydroxyd-Lösung auf pH 11,6 gebracht. Die Acetoxy-Funktion wird nun hydrolysiert. Durch laufenden Zusatz von NaOH wird das pH konstant gehalten. Insgesamt werden 29 ml 2N NaOH verbraucht.

Die erhaltene alkalische Lösung wird mit 200 ml Wasser verdünnt und durch Perkolation durch eine mit Kationen austauscherharz (z.B. Amberlite(R) IR-120) und eine mit Anionenaustauscherharz (z.B. Amberlite(R) IR-45) beschickte Säule entsalzt.

Das Säuleneluat wird zur Trockene eingedampft.

Ausbeute : 11,08 g L-5-(N-Methyl-α-hydroxypropionyl-amino)-2,4,6-trijod-isophthalsäure-bis-(1,3-dihydroxyisopropylamid), das sind 70 % der Theorie.

Schmelzpunkt (nach wiederholtem Umkristallisieren aus abs. Äthanol) > 280 °C (sintern bei 210 °C).

DC auf Kieselgel : Laufmittel Aethylacetat/Eisessig/Wasser = 10 : 5 : 3. Ein Flecken bei Rf 0,29.

Das als Zwischenprodukt verwendete L-5-(N-Methyl-α-acetoypropionyl-amino)-2,4,6-trijod-isophthalsäuredichlorid wird wie folgt erhalten :

A. 5-Amino-2,4,6-trijod-isophthalsäure wird nach der Methode, die in der DE-OS 2 050 217 beschrieben ist, in Schwefelsäure mit Formaldehyd behandelt, wobei 5-Methylamino-2,4,6-trijod-isophthalsäure vom Schmelzpunkt 198-200 °C erhalten wird.

DC auf Kieselgel mit Äthylmethylketon/Äthanol/Wasser/Eisessig = 20 : 8 : 5 : 1,5. Rf 0,55.

B. 23 g 5-Methylamino-2,4,6-trijod-isophthalsäure werden in 120 ml Thionylchlorid in Gegenwart von 0,1 ml Chinolin während 7 Stunden am Rückfluss gekocht. Nach dem vollständigen Abdestillieren des Thionylchlorids wird der Rückstand in 120 g Eiswasser, das Kochsalz (125 g) und $NaHCO_3$ (12 g) enthält, eingerührt.

Das Produkt wird mit Äthylacetat (200 ml) extrahiert. Aus dem Extrakt wird durch Einengen 5-Methylamino-2,4,6-trijod-isophthalsäure-dichlorid erhalten.

Schmelzpunkt 167 °C. DC auf Kieselgel mit Benzol/Hexan = 1 : 1 ; Rf 0,50.

$C_9H_4Cl_2J_3NO_2$ :

Cl ber. 11,62 % Cl gef. 11,74 %.
J ber. 62,44 % J gef. 62,74 %.

C. 12 g 5-Methylamino-2,4,6-trijod-isophthalsäure-dichlorid (0,02 Mol) werden in 30 ml DMAC tropfenweise bei 0 bis 2 °C mit L-α-Acetoxy-propionsäurechlorid (0,03 Mol) versetzt. Anschliessend wird noch 1 bis 2 Stunden bei 20 °C gerührt.

Die Reaktionslösung wird in Eiswasser eingerührt. Das ausgeschiedene Produkt wird abfiltriert, getrocknet und aus wenig Benzol umkristallisiert.

Man erhält so 14 g L-5-(N-Methyl-α-acetoxypropionyl-amino)-2,4,6-trijod-isophthalsäuredichlorid vom Schmelzpunkt 187-190 °C.

DC auf Kieselgel mit Hexan/Chloroform/Äthylacetat = 3 : 1 : 1 ; 2 Flecken Rf 0,22 und 0,5.

$C_{14}H_{10}Cl_2J_3NO_5$ :

Cl ber. 9,79 % Cl gef. 9,80 %.
J ber. 52,59 % J gef. 52,46 %.

## Beispiel 8

L-5-(N-Methyl-α-hydroxypropionyl-amino)-2,4,6-trijodisophthalsäure-bis-(2,3-dihydroxypropylamid)

14,5 g L-5-(N-Methyl-α-acetoxypropionyl-amino)-2,4,6-trijod-isophthalsäure-dichlorid (0,02 Mol) in 30 ml DMF werden tropfenweise mit 9,4 g 2,3-Dihydroxypropylamin (= 1-Amino-2,3-propandiol) gelöst, in 50 ml DMF versetzt und nach der im Beispiel 7 beschriebenen Methode umgesetzt und aufgearbeitet.

Man erhält 10,8 g L-5-(N-Methyl-α-hydroxypropionyl-amino)-2,4,6-trijod-isophthalsäure-bis-(2,3-dihydroxypropylamid), das sind 68 % der Theorie.

Schmelzpunkt (nach Umkristallisation aus Äthanol) 195 °C (sintern bei 187 °C).

DC auf Kieselgel : Laufmitttel Äthylacetat/Eisessig/Wasser = 10 : 5 : 3. Ein Flecken bei Rf 0,45.

$C_{18}H_{24}J_3N_3O_8 \cdot H_2O$ :

J ber. 47,05 % gef. 47,00 %.
$H_2O$ ber. 2,23 % gef. 2,8 %.

## Beispiel 9

5-(N-Methyl-hydroxyacetyl-amino)-2,4,6-trijod-isophthalsäure-bis-(2,3-dihydroxypropylamid)

Analog Beispiel 1 hergestellte 5-N-Natrium-Verbindung von 5-Hydroxyacetylamino-2,4,6-trijod-isophthalsäure-bis-(2,3-dihydroxypropylamid) (49 g = 0,062 5 Mol) in 250 ml DMAC werden bei 5 °C tropfenweise mit 13,5 g Methyljodid (0,095 Mol) versetzt und anschliessend noch einige Stunden gerührt.

Die Reaktionslösung wird im Vakuum eingeengt, der Eindampfrückstand wird mit 300 ml Methylenchlorid versetzt worauf das gebildete Produkt vermischt mit Natriumjodid ausgeschieden wird. Das rohe Produkt wird in Wasser gelöst und mit Ionenaustauscherharz entsalzt.

Man erhält 36 g 5-(N-Methyl-hydroxyacetyl-amino)-2,4,6-trijod-isophthalsäure-bis-(2,3-dihydroxypropylamid), das sind 75 % der Theorie.

Schmelzpunkt : 190-191 °C (amorphes Produkt).

DC auf Kieselgel : Laufmittel 2-Butanon/Eisessig/Wasser = 15 : 3 : 5.

Flecken bei Rf 0,48 und 0,40.

Löslichkeiten : Spielend leicht löslich in Wasser und Methanol. Nur beschränkt löslich in Äthanol (in 20 Vol. teilen bei Siedehitze und in 35 Vol. teilen bei 25 °C).

Das als Zwischenprodukt verwendete 5-Hydroxyacetylamino-2,4,6-trijod-isophthalsäure-bis-(2,3-dihydroxypropylamid) wird nach der in der DE-PS 2 457 789 beschriebenen Methode wie folgt erhalten :

24,4 g 5-Acetoxyacetylamino-2,4,6-trijod-isophthalsäuredichlorid (0,035 Mol) gelöst in 60 ml DMAC werden unter Rühren zu einer Lösung von 15,9 g 2,3-Dihydroxypropylamin [= 1-Amino-2,3-dihydroxy-propan] (0,175 Mol) in 100 ml DMAC getropft.

Man erhält oeliges 5-Acetoxyacetylamino-2,4,6-trijodisophthalsäure-bis-(2,3-dihydroxypropylamid). Diese Verbindung wird in 250 ml Wasser aufgenommen und bei 40 °C vorsichtig mit 40 ml 1N NaOH behandelt bis die Acetoxy-Gruppe vollständig hydrolysiert ist.

Die erhaltene Lösung wird durch Perkolation durch eine mit Kationenaustauscherharz (Amberlite(R) IR 120) und eine mit Anionenaustauscherharz (Amberlite(R) IR 45) beschickte Säule entsalzt. Das Eluat wird eingedampft. Nach einiger Zeit tritt Kristallisation ein. Durch Umkristallisieren aus wenig Wasser wird das gewünschte Zwischenprodukt 5-Hydroxyacetylamino-2,4,6-trijod-isophthalsäure-bis-(2,3-dihydroxypropylamid) (19,4 g) in reiner Form erhalten.

Schmelzpunkt : 290 °C.

DC : Rf = 0,24 ; Laufmittel : Äthylacetat/Äthanol/Ammoniak (25 %ig) = 15 : 7 : 6.

$C_{16}H_{20}J_3N_3O_8$ :

C ber. 25,18 % gef. 25,01 %.

J ber. 49,89 % gef. 49,75 %.

## Beispiel 10

5-(N-Methyl-hydroxyacetyl-amino)-2,4,6-trijod-isophthalsäure-bis-(2,3-dihydroxypropylamid)

A. 90 g 5-Hydroxyacetylamino-2,4,6-trijod-isophthalsäure-bis-(2,3-dihydroxypropylamid) (0,117 Mol) werden in 700 ml DMAC suspendiert und bei 40 °C mit 95 g einer Lösung von NaOH in Methanol (1,233 Mol) versetzt. Es bildet sich die 5-N-Natrium-Verbindung. Methanol, Reaktionswasser und ein Teil des DMAC werden im Vakuum abdestilliert. Man erhält 496 g einer Lösung enthaltend 0,234 Mol 5-N-Na-Verbindung von 5-Hydroxyacetylamino-2,4,6-trijod-isophthalsäure-bis-(2,3-dihydroxypropylamid).

B. Zu einer Lösung von 13 g Methylbromid (0,137 Mol) in 160 g DMAC werden bei 0 °C unter Rühren innert 45 Minuten 390 g (0,091 Mol) der im Abschnitt A. beschriebenen Lösung eingetropft und anschliessend noch einige Stunden bei 0 bis 5 °C gerührt.

Die Aufarbeitung erfolgt nach der im Beispiel 9 beschriebenen Methode.

Man erhält : 60,1 g 5-(N-Methyl-hydroxyacetyl-amino)-2,4,6-trijod-isophthalsäure-bis-(2,3-dihydroxy-propylamid) d.s. 85 % der Theorie.

DC auf Kieselgel mit Laufmittel Äthylacetat/Eisessig/Wasser = 10 : 5 : 3. Flecken bei Rf 0,3 und 0,45. Das Produkt lässt sich aus 95 %igem Äthanol umkristallisieren.

Schmelzpunkt : 305-310 °C unter Zersetzung.

Der Schmelzpunkt ist nicht sehr charakteristisch.

C. Zu einer Lösung von 13,8 g Dimethylsulfat (0,109 Mol) in 150 ml DMAC werden unter Rühren innert 50 Minuten 390 g (0,091 Mol) der im Abschnitt A beschriebenen Lösung der Na-Verbindung von 5-Hydroxyacetylamino-2,4,6-trijod-isophthalsäure-bis-(2,3-dihydroxypropylamid) eingetropft. Die Reaktionslösung wird einige Stunden gerührt und anschliessend nach der im Beispiel 1 beschriebenen Methode aufgearbeitet.

Man erhält : 62,4 g 5-(N-Methyl-hydroxyacetyl-amino)-2,4,6-trijod-isophthalsäure-bis-(2,3-dihydroxy-propylamid) das sind 88 % der Theorie.

Schmelzpunkt (nach Umkristallisieren aus 95 %igem Äthanol) : 305-310 °C unter Zersetzung.

## Beispiel 11

5-(N-Methyl-hydroxyacetyl-amino)-2,4,6-trijod-isophthalsäure-bis-(2,3-dihydroxypropylamid)
Alternative Synthese

In eine Lösung von 18,2 g 1-Amino-2,3-propandiol in 70 ml DMAC werden bei 5 °C unter Rühren innert 45′ 28,4 g 5-(N-Methyl-acetoxyacetyl-amino)-2,4,6-trijod-isophthalsäuredichlorid in 90 ml DMAC getropft. Die Reaktionsmischung wird noch einige Stunden gerührt und anschliessend im Vakuum bis zum Sirup eingedampft. Der Rückstand wird mit Methylenchlorid und mit Aceton verrieben, das Lösungsmittel wird abdekantiert, der Rückstand wird durch Evakuieren von anhaftendem Lösungsmittel befreit, in 200 ml Wasser aufgenommen und bei 45 °C vorsichtig durch Zusatz von insgesamt 50 ml 2N Natriumhydroxyd-Lösung bei pH 11 bis 11,5 gehalten, wobei die Acetoxy-Gruppe hydrolysiert wird.

Die erhaltene Lösung wird durch Perkolation durch eine mit Kationenaustauscherharz (z.B. 200 ml Amberlite(R) IR-120) und eine zweite mit Anionenaustauscherharz (z.B. 250 ml Amberlite(R) IR-45) beschickte Säule entsalzt.

Das Eluat wird eingedampft, der Rückstand wird in Methanol gelöst, die Lösung mit Methylenchlorid versetzt wobei das gewünschte Produkt ausfällt.

Ausbeute : 22 g der Titelverbindung, das sind 71 % der Theorie.

Schmelzpunkt : ca. 190 °C (sintern bei ca. 165 °C).

DC auf Kieselgel : Laufmittel 2-Butanon/Eisessig/Wasser = 15 : 3 : 5. Flecken bei Rf 0,48 und 0,40.

$C_{17}H_{22}J_3N_3O_8$ : J ber. 48,99 % gef. 48,69 %.

Die Verbindung ist spielend leicht löslich in Wasser (3 g in 1 ml Wasser) und in Methanol (100 % g/v).

Das als Zwischenprodukt verwendete 5-(N-Methyl-acetoxy-acetyl-amino)-2,4,6-trijod-isophthalsäure-dichlorid wird wie folgt erhalten :

32 g 5-Methylamino-2,4,6-trijod-isophthalsäure-dichlorid (0,0525 Mol) in 80 ml DMAC werden unter Rühren bei 0 bis 5 °C tropfenweise mit 10,7 g Acetoxyacetylchlorid versetzt.

Anschliessend wird noch über Nacht bei Raumtemperatur gerührt. Die Reaktionslösung wird in Eiswasser eingerührt. Man erhält 36,7 g 5-(N-Methyl-acetoxyacetyl-amino)-2,4,6-trijod-isophthalsäure-dichlorid vom Schmelzpunkt 198-200 °C. Ausbeute : 98,8 % der Theorie.

DC auf Kieselgel mit Benzol/Methanol = 10 : 3 ; Rf = 0,64.

$C_{13}H_8Cl_2J_3NO_5$ :
ber. Cl 9,9 % J 53,3 %
gef. Cl 10,05 % J 53,41 %.

## Beispiel 12

5-(N-Methyl-hydroxyacetyl-amino)-2,4,6-trijod-isophthalsäure-bis-(R(+)2,3-dihydroxypropylamid)

Diese Verbindung wird wie die entsprechende racemische Verbindung erhalten, indem man ähnlich wie im Beispiel 11 beschrieben in eine Lösung von 7 g R(+)1-Amino-2,3-propandiol (0,077 Mol) in 40 ml DMAC, in welcher 10,8 g Kaliumcarbonat (0,077 Mol) suspendiert sind, 20,2 g 5-(N-Methyl-acetoxyacetyl-amino)-2,4,6-trijod-isophthalsäure-dichlorid gelöst in 40 ml DMAC eintropft, die Reaktionsmischung einige Stunden rührt und anschliessend wie im Beispiel 10 beschrieben aufarbeitet.

Man erhält 15,2 g der Titelverbindung, das sind 69,5 % der Theorie.

Schmelzpunkt : 283-284 °C.

DC : Rf = 0,24. Laufmittel Isopropanol/Isobutanol/Ammoniak 25 %ig = 7 : 7 : 6.

$C_{17}H_{22}J_3N_3O_8$ : J ber. 48,99 % gef. 48,74 %.

$[\alpha]_D^{20} = + 4,85°$, $[\alpha]_{436}^{20} = + 11,1°$ (c = 10 % in Wasser).

## Beispiel 13

5-(N-Methyl-hydroxyacetyl-amino)-2,4,6-trijod-isophthalsäure-bis-(2,3-dihydroxypropylamid)

A. 5-(N-Methyl-hydroxyacetyl-amino)-2,4,6-trijod-isophthalsäure-bis-(2,3-isopropylidendioxypropyl-amid) = 5-(N-Methyl-hydroxyacetyl-amino)-2,4,6-trijod-isophthalsäure-bis-(2,2-dimethyl-1,3-dioxolan-(4)-ylmethyl-amid).

17,8 g 5-(N-Methyl-acetoxyacetyl-amino)-2,4,6-trijodisophthalsäure-dichlorid (0,025 Mol) in 50 ml DMAC werden bei 5-8 °C unter Rühren tropfenweise mit einer Lösung von 16 g 4-Aminomethyl-2,2-dimethyl-1,3-dioxolan (0,122 Mol) versetzt. Man rührt 18 Stunden bei Raumtemperatur, filtriert das ausgefallene Hydrochlorid ab und dampft das Filtrat im Vakuum vollständig ein. Der Eindampfrückstand wird in Wasser suspendiert, abfiltriert, in wässrigem Methanol gelöst und bei 50-55 °C mit 2N NaOH bei pH 10,5 bis 11,0 behandelt, wobei die Acetoxygruppe vollständig hydrolysiert wird. Die erhaltene Lösung wird durch vorsichtigen Zusatz von Salzsäure exakt neutralisiert, klarfiltriert und zur Trockene eingedampft. Der Rückstand wird in Wasser aufgenommen, wobei das erhaltene 5-(N-Methyl-hydroxyacetyl-amino)-2,4,6-trijod-isophthalsäure-bis-(2,3-isopropylidendioxypropylamid) kristallisiert.

Ausbeute : 15,2 g, das sind 71 % der Theorie.

Schmelzpunkt : (nach Umkristallisieren aus verdünntem Methanol) 180-181 °C.

DC : Rf = 0,295, Laufmittel Chloroform/Hexan/Methanol = 3 : 3 : 1.

$C_{23}H_{30}J_3N_3O_8$ : J ber. 44,41 % gef. 44,08 %.

Diese Verbindung ist sehr leicht löslich in Methanol, Äthanol und Chloroform, dagegen nur wenig löslich in Wasser.

B. 5-(N-Methyl-hydroxyacetyl-amino)-2,4,6-trijod-isophthalsäure-bis-(2,3-dihydroxypropylamid).

15 g 5-(N-Methyl-hydroxyacetyl-amino)-2,4,6-trijod-isophthalsäure-bis-(2,3-isopropylidendioxypropylamid) in einer Lösung aus 185 ml 0,1N wässriger Salzsäure und 185 ml Methanol gelöst und unter Rühren während 5 Stunden auf 50 °C gehalten. Die Reaktionslösung wird durch Perkolation durch eine mit 75 ml einem schwach basischen Ionenaustauscherharz z.B. Amberlite(R) IR-45 beschickte Säule von Salzsäure befreit und zur Trockene eingedampft.

Man erhält 12,2 g 5-(N-Methyl-hydroxyacetyl-amino)-2,4,6-trijod-isophthalsäure-bis-(2,3-dihydroxy-propylamid) d.s. 90 % der Theorie.

Schmelzpunkt : 190 °C (amorphes Produkt).

Nach umkristallisieren aus 95 %igem Äthanol.

Schmelzpunkt : 300 °C unter Zersetzung.

Verwendung :

Unter den in den vorstehenden Beispielen beschriebenen Verbindungen werden im allgemeinen die 5-(N-Methyl-α-hydroxyacyl-amino)-2,4,6-trijod-isophthalsäure-bis-(hydroxyalkylamide) der Formel (II) bevorzugt, da sie sowohl besser wasserlöslich als auch leichter zugänglich sind als die höheren N-Alkyl-Derivate davon.

Innerhalb den bevorzugten Verbindungen der Formel (II) werden die Hydroxyacetyl-Derivate gewöhnlich den α-Hydroxypropionyl-Derivaten vorgezogen, weil sie einfacher herzustellen sind, kein Chiralitätszentrum aufweisen und trotzdem im allgemeinen die geforderte hohe Wasserlöslichkeit besitzen.

Wegen ihrer einfachen Herstellung werden unter den Hydroxyalkylamiden die 2,3-Dihydroxypropylamide vorgezogen. Ein typischer Vertreter dieser Gruppe ist das 5-(N-Methyl-hydroxyacetylamino)-2,4,6-trijod-isophthalsäure-bis-(2,3-dihydroxypropylamid (Verbindung) A. Diese Verbindung zeichnet sich durch vergleichsweise besonders hohe Wasserlöslichkeit, niedrige Viskosität ihrer wässrigen Lösungen und durch hohe Stabilität aus.

In den folgenden Tabellen sind wichtige Eigenschaften von Verbindung A mit zwei vorbekannten nicht-ionogenen Röntgenkontrastmitteln verglichen, und zwar mit

B : L-5-α-Hydroxypropionylamino-2,4,6-trijod-isophthalsäure-bis-(1,3-dihydroxyisopropylamid) (Freiname [international non-proprietory name I.N.N.] = IOPAMIDOL) und

C : 3-Acetylamino-4-N-methyl-acetylamino-2,4,6-trijod-benzoyl-glucosamin (Freiname [I.N.N.] = METRIZAMIDE)

TABELLE 1

| Verbindung | Löslichkeit in Wasser i. % (g/v) bei 20 °C | Viskosität in Centipoise (cP) wässrige Lösungen enthaltend °C | 300 mg J/ml | 400 mg J/ml |
|---|---|---|---|---|
| | | 20 °C | 7,55 | 22,0 |
| A | > 100 | 37 °C | 4,19 | 9,87 |
| | | 20 °C | 8,95 | 40,6 |
| B | 89 | 37 °C | 4,70 | 16,1 |
| | | 20 °C | 11,7 | 77,8 |
| C | ~ 80 | 37 °C | 5,98 | 26,9 |

TABELLE 2

| Verbindung | Mg J/ml | Osmolalität (mOsm/kg) 37 °C | osmotischer Druck atm 37 °C |
|---|---|---|---|
| A | 250 | 452 | 11,52 |
| | 300 | 536 | 13,64 |
| | 350 | 628 | 15,98 |
| B | 250 | 514 | 13,09 |
| | 300 | 619 | 15,76 |
| | 350 | 737 | 18,77 |

Aus der Tabelle 1 geht klar hervor, dass die erfindungsgemäss erhaltene Verbindung A eine höhere Wasserlöslichkeit und beträchtlich niedrigere Viskosität aufweist als die vorbekannten Vergleichsverbindungen B und C. Lösungen von A können daher in höherer Konzentration angewandt werden und lassen sich dank ihrer niedrigen Viskosität trotzdem ohne Schwierigkeiten injizieren.

Aus Tabelle 2 ist ersichtlich, dass der osmotische Druck der erfindungsgemäss erhaltenen Verbindung A geringer ist als der von IOPAMIDOL. Die Belastung des Organismus bei Verabreichung von Verbindung A ist daher geringer als nach Verabreichung von Verbindung B.

Die neuen 5-(N-Alkyl-α-hydroxyacyl-amino)-2,4,6-trijodisophthalsäure-bis-(hydroxyalkyl-amide) der allgemeinen Formel (I) werden vorwiegend in Form ihrer wässrigen Lösungen verwendet.

Es kommen je nach Verwendungszweck ca. 15 — bis 85 %ige Lösungen — g/v, d.h. 100 %ig = 100 g

Kontrastmittel/100 ml Lösung — mit einem Gehalt von etwa 60 bis ca. 420 mgJ/ml zur Anwendung. Konzentrierte Lösungen werden bevorzugt. Die Art ihrer Applikation richtet sich nach dem sichtbar zu machenden Organ.

Für die Vasographie werden die Lösungen in die entsprechenden Blutgefässe injiziert oder infundiert.

Für die Urographie werden die Lösungen intravenös injiziert oder infundiert.

Für die Kontrastverstärkung in der Computertomographie werden die Lösungen je nach zu verstärkendem Organ- oder Gewebekontrast entweder durch intravenöse Verabreichung in den Blutkreislauf eingeführt oder durch selektive Injektion im Gefässsystem eines einzelnen Organs oder einer Körperhöhle angereichert.

Für die Myelographie und Radiculographie werden die Lösungen nach lumbaler oder subokzipitaler Punktion instilliert. Bei der Ventriculographie werden direkt die Ventrikel punktiert.

Dosierung :

| | |
|---|---|
| Myelographie | ca. 5-15 ml |
| Radiculographie | ca. 3- 5 ml |
| Ventriculographie | ca. 1- 2 ml |

Die Herstellung der Röntgenkontrastmittellösungen ist einfach, weil keine Salzlösungen bereitet werden müssen.

Beispielsweise werden die nach den vorstehenden Beispielen erhaltenen reinen 2,4,6-Trijod-isophthalsäureamide unter sterilen Bedingungen in der gewünschten Menge bidestilliertem Wasser gelöst, filtriert, in Serumflaschen oder Ampullen abgefüllt und anschliessend sterilisiert. Die vorliegenden Trijod-isophthalsäureamide werden beim Hitzesterilisieren nicht zersetzt.

Beispiel 14

Injektionslösungen enthaltend 5-(N-Methyl-hydroxyacetyl-amino)-2,4,6-trijod-isophthalsäure-bis-(2,3-dihydroxypropylamid) = Verbindung A

| Zusammensetzung von jeweils 20 ml Lösung | | Gehalt der Injektionslösungen in mgJ/ml 200 | 300 | 420 |
|---|---|---|---|---|
| Verbindung A | g | 8,16 | 12,25 | 17,15 |
| Di-Na-Ca-Salz von Äthylendiamin-tetra-essigsäurehexahydrat | mg | 5,2 | 7,8 | 11 |
| Tromethamin [Tris-(hydroxy-methyl)-amino-methan] | mg | 9,5 | 14,2 | 20 |
| Bidestilliertes Wasser bis | ml | 20 | 20 | 20 |
| Dichte bei 37 °C | d | 1,207 | 1,316 | 1,453 |
| Viskosität bei 37 °C (cP = Centipoise) | cP | 1,87 | 4,19 | 20,03 |

Ausführung : Das Na-Ca-Salz von Äthylendiamintetraessigsäure, das Tromethamin und das Kontrastmittel werden in bidestilliertem Wasser gelöst. Das pH der Lösung wird nötigenfalls durch Zusatz von 1N Salzsäure auf ca. 7 eingestellt. Das Volumen wird auf 20 ml aufgefüllt. Die Lösung wird filtriert unter Verwendung einer Membrane von 0,45 mμ. Das Filtrat wird in Ampullen abgefüllt und während 30 Minuten bei 120 °C sterilisiert.

Beispiel 15

Injektionslösung

| | |
|---|---|
| 5-(N-Methyl-hydroxyacetyl-amino)-2,4,6-trijod-isophthalsäure-bis-(1,3-dihydroxy-isopropylamid) | 82 g |
| 5-(N-Methyl-hydroxyacetyl-amino)-2,4,6-trijod-isophthalsäure-bis-(2,3-dihydroxy-propylamid) | 20,5 g |
| Natriumcarbonat | 0,4 g |
| Dinatriumsalz von Äthylendiamintetraessigsäure | 0,02 g |
| Bidestilliertes Wasser bis zum Volumen von | 125 ml |

Ausführung : Die Komponenten werden vereinigt mit bidestilliertem Wasser auf 125 ml aufgefüllt, filtriert, unter hygienisch einwandfreien Bedingungen unter Stickstoff in Ampullen abgefüllt und anschliessend sterilisiert. Jodgehalt : 400 mg/ml.

Beispiel 16

Infusionslösung

| | |
|---|---|
| 5-(N-Methyl-α-hydroxypropionylamino)-2,4,6-trijod-isophthalsäure-bis-(1,3-dihydroxy-isopropylamid) | 155,9 g |
| Dinatriumsalz von Äthylendiamintetraessigsäure | 0,02 g |
| Bidestilliertes Wasser bis zum Volumen von | 250 ml |

Ausführung : Die Komponenten werden vereinigt auf 250 ml aufgefüllt, unter Stickstoff in eine Infusionsflasche abgefüllt und sterilisiert. Jodgehalt : 300 mg/ml.

**Ansprüche**

1. 5-(N-Alkyl-α-hydroxyacyl-amino)-2,4,6-trijod-isophthalsäure-bis-(hydroxyalkylamide) der allgemeinen Formel (I)

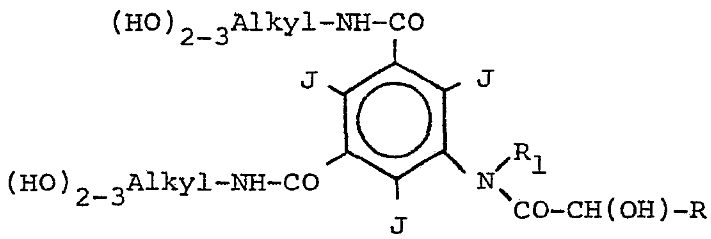

(I),

worin
$(HO)_{2-3}$Alkyl-1,3-Dihydroxyisopropyl, 2,3-Dihydroxypropyl- oder 1,3-Dihydroxy-2-hydroxymethyl-isopropyl-,
R Wasserstoff oder Methyl- und
$R_1$ einen Alkyl-Rest mit 1 bis 5 C-Atomen bedeutet.

2. 5-(N-Methyl-α-hydroxyacyl-amino)-2,4,6-trijod-isophthalsäure-bis-(hydroxyalkylamide) der allgemeinen Formel (II)

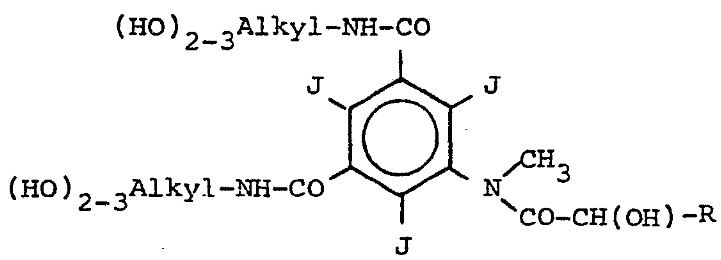

(II),

worin
$(HO)_{2-3}$Alkyl-1,3-Dihydroxyisopropyl-, 2,3-Dihydroxypropyl- oder 1,3-Dihydroxy-2-hydroxymethyl-isopropyl- und
R Wasserstoff oder Methyl bedeutet.

3. 5-(N-Methyl-hydroxyacetyl-amino)-2,4,6-trijod-isophthalsäure-bis-(2,3-dihydroxypropylamid).

4. 5-(N-Methyl-α-hydroxypropionyl-amino)-2,4,6-trijodisophthalsäure-bis-(1,3-dihydroxyisopropylamid).

5. Verfahren zur Herstellung der als schattengebende Komponenten in Röntgenkontrastmitteln verwendbaren 5-(N-Alkyl-α-hydroxyacyl-amino)-2,4,6-trijod-isophthalsäure-bis-(hydroxyalkylamide) nach Anspruch 1, dadurch gekennzeichnet, dass man ein 5-α-Hydroxyacylamino-2,4,6-trijod-isophthalsäure-bis-(hydroxyalkylamid) der allgemeinen Formel (III)

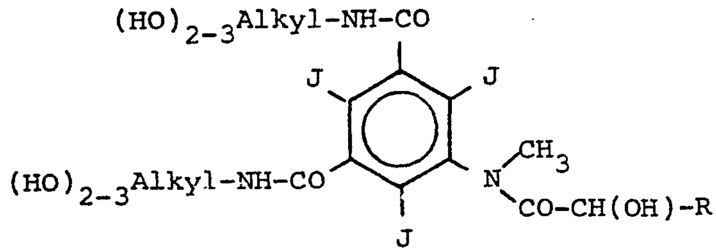

(III),

am aromatischen Stickstoff-Atom im alkalischen Milieu alkyliert durch Umsetzung mit Alkylierungsmitteln der allgemeinen Formel (IV)

$$R_1—X \quad (IV),$$

wobei R und $R_1$ in den Formeln (III) bzw. (IV) die im Anspruch 1 definierte Bedeutung haben und X eines der Halogen-Atome Jod, Brom oder Chlor oder ein Sulfat- bzw. Sulfonat-Radikal ($—OSO_2—OR_1$ bzw. $—OSO_2$-Alkyl oder $—OSO_2$-Aryl) bedeutet, oder dass man ein reaktives funktionelles Derivat einer 5-(N-Alkyl-α-hydroxyacylamino)-2,4,6-trijod-isophthalsäure der allgemeinen Formel (V)

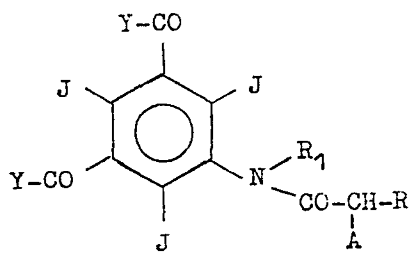

(V),

worin R und $R_1$ die im Anspruch 1 definierte Bedeutung haben, A einen niedrigen Acyloxy-Rest mit 1 bis 5 C-Atomen oder ein Halogen-Atom und Y—CO— reaktive Säurehalogenid oder -anhydrid-Radikale bedeuten, mit einem Di- oder Trihydroxyalkylamin $(HO)_{2-3}$Alkyl-$NH_2$, dessen Hydroxy-Funktionen durch Acetalisierung oder Ketalisierung maskiert sein können, umsetzt und im erhaltenen 5-(N-Alkyl-α-hydroxyacyl-amino)-2,4,6-trijod-isophthalsäure-bis-(hydroxyalkylamid)-Derivat die maskierende Funktion A und gegebenenfalls vorhandene Acetal- oder Ketal-Funktionen hydrolytisch in die Hydroxy-Funktionen spaltet.

6. Röntgenkonstrastmittel, dadurch gekennzeichnet, dass sie als schattengebende Komponenten mindestens ein 5-(N-Alkyl-α-hydroxyacyl-amino)-2,4,6-trijod-isophthalsäure-bis-(hydroxyalkylamid) gemäss Anspruch 1 enthalten.

**Claims**

1. 5-(N-alkyl-α-hydroxyacyl-amino)-2,4,6-triiodo-isophthalic-bis-(hydroxyalkylamides) of the general formula (I)

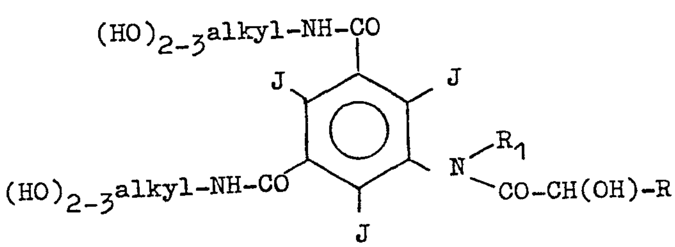

(I),

wherein

$(HO)_{2-3}$alkyl- connotes 1,3-dihydroxyisopropyl, 2,3-dihydroxypropyl- or 1,3-dihydroxy-2-hydroxymethyl-isopropyl-,

R connotes hydrogen or methyl- and

$R_1$ connotes an alkyl radical with 1 to 5 C-atoms.

2. 5-(N-methyl-α-hydroxyacyl-amino)-2,4,6-triiodo-isophthalic-bis-(hydroxyalkylamides) of the general formula (II)

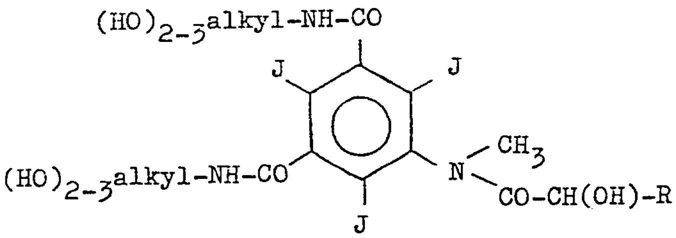

(II),

wherein

$(HO)_{2-3}$alkyl- connotes 1,3-dihydroxyisopropyl-, 2,3-dihydroxypropyl- or 1,3-dihydroxy-2-hydroxymethyl-isopropyl- and

R connotes hydrogen or methyl.

3. 5-(N-methyl-hydroxyacetyl-amino)-2,4,6-triiodo-isophthalic-bis-(2,3-dihydroxypropylamide).

4. 5-(N-methyl-α-hydroxypropionyl-amino)-2,4,6-triiodo-isophthalic-bis-(1,3-dihydroxyisopropylamide).

5. Method of producing the 5-(N-alkyl-α-hydroxyacylamino)-2,4,6-triiodo-isophthalic-bis-(hydroxyalkylamides) useful as shading components in X-ray contrast media according to Claim 1, characterised in that a 5-α-hydroxyacylamino-2,4,6-triiodo-isophthalic-bis-(hydroxyalkylamide) of the general formula (III)

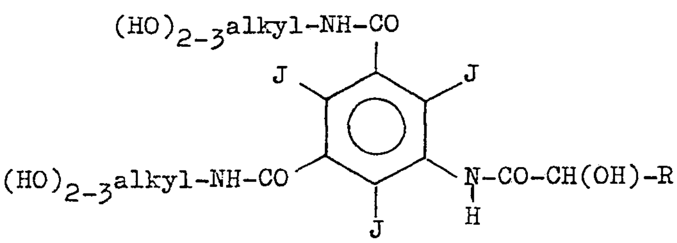

(III),

is alkylated at the aromatic nitrogen atom in an alkaline medium by a reaction with alkylating agent of the general formula (IV)

$$R_1—X \qquad (IV),$$

wherein R and $R_1$ in formulae (III) and (IV) have the connotation defined in Claim 1 and X connotes one of the halogen atoms iodine, bromine or chlorine or a sulphate or sulphonate radical ($—OSO_2—OR_1$ or $—OSO_2$-alkyl or $—OSO_2$-aryl) or that a reactive functional derivative of a 5-(N-alkyl-α-hydroxyacyl-amino)-2,4,6-triiodo-isophthalic acid of the general formula (V)

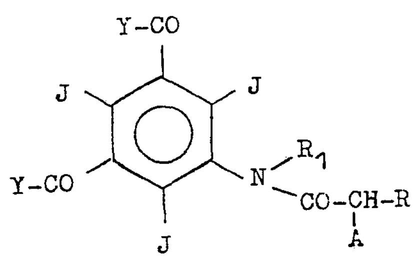

(V),

wherein R and $R_1$ have the connotation defined in Claim 1, A connotes a low acyloxy radical with from 1 to 5 C-atoms or a halogen atom and Y—CO— connotes reactive acid halide or anhydride radicals, is reacted with a di- or tri- hydroxyalkylamine $(HO)_{2-3}alkyl-NH_2$, the hydroxy functions of which may be masked by acetalisation or ketalisation and in the 5-(N-alkyl-α-hydroxyacyl-amino)-2,4,6-triiodo-isophthalic-bis-(hydroxyalkylamide) derivative obtained the masking function A and acetal or ketal functions possibly present are split hydrolytically into the hydroxy functions.

6. X-ray contrast media, characterised in that they comprise as shading components at least one 5-(N-alkyl-α-hydroxyacyl-amino)-2,4,6-triiodo-isophthalic-bis-(hydroxyalkylamide) according to Claim 1.

**Revendications**

1. Bis-(hydroxyalkylamides) d'acides 5-(N-alkyl-α-hydroxyacyl-amino)-2,4,6-triiodo-isophtaliques de formule générale I

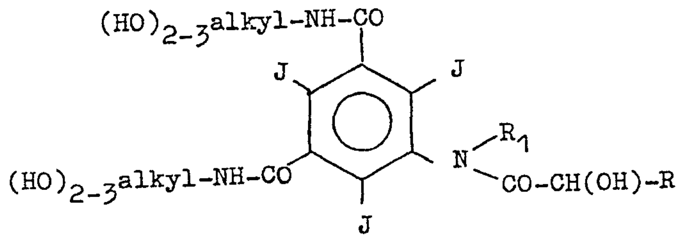

(I)

dans laquelle

$(HO)_{2-3}$alkyl- représente un groupe 1,3-dihydroxyisopropyle, 2,3-dihydroxypropyle ou 1,3-dihydroxy-2-hydroxyméthyl-isopropyle,

R représente l'hydrogène ou un groupe méthyle et

$R_1$ représente un groupe alkyle en C1-C5

2. Bis-(hydroxyalkylamides) d'acides 5-(N-méthyl-α-hydroxyacylamino)-2,4,6-triiodo-isophtaliques de formule générale II

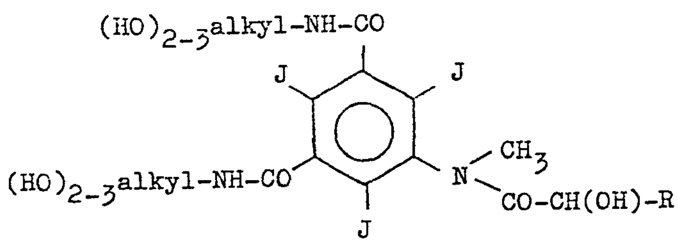

(II)

dans laquelle

$(HO)_{2-3}$alkyl- représente un groupe 1,3-dihydroxyisopropyle, 2,3-dihydroxypropyle ou 1,3-dihydroxy-2-hydroxyméthyl-isopropyle et

R représente l'hydrogène ou un groupe méthyle.

3. Le bis-(2,3-dihydroxypropylamide) de l'acide 5-(N-méthyl-hydroxyacétyl-amino)-2,4,6-triiodo-isophtalique.

4. Le bis-(1,3-dihydroxyisopropylamide) de l'acide 5-(N-méthyl-α-hydroxypropionyl-amino)-2,4,6-triiodo-isophtalique.

5. Procédé de préparation des bis-(hydroxyalkylamides) d'acides 5-(N-alkyl-α-hydroxyacyl-amino)-2,4,6-triiodo-isophtaliques selon la revendication 1, utilisables en tant que composants opacifiants dans des agents de contraste aux rayons X, caractérisé en ce que l'on alkyle en milieu alcalin sur l'atome d'azote aromatique un bis-(hydroxyalkylamide) d'acide 5-α-hydroxyacylamino-2,4,6-triiodo-isophtalique de formule générale III

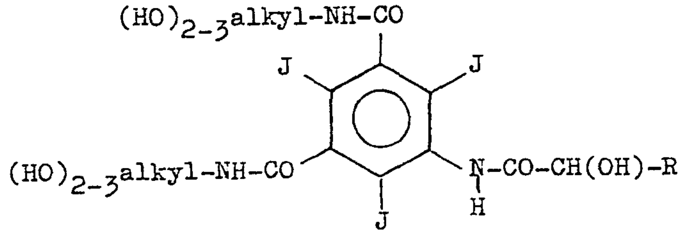

(III)

par réaction avec des agents alkylants de formule générale IV

$$R_1—X \qquad (IV)$$

R et $R_1$ des formules III et IV ayant les significations indiquées dans la revendication 1 et X représentant l'un des atomes d'halogènes iode, brome ou chlore, ou un radical sulfate ou sulfonate ($—OSO_2—OR_1$ ou

—$OSO_2$-alkyle ou —$OSO_2$-aryle), ou bien en ce que l'on fait réagir un dérivé fonctionnel réactif d'un acide 5-(N-alkyl-α-hydroxyacyl-amino)-2,4,6-triiodo-isophtalique de formule générale V

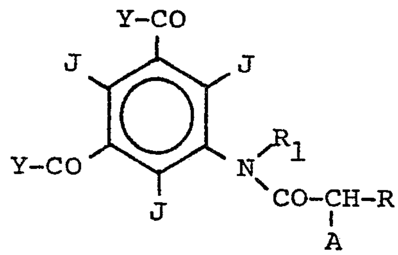

(V)

dans laquelle R et $R_1$ ont les significations indiquées dans la revendication 1, A représente un groupe acyloxy inférieur en C1-C5 ou un atome d'halogène et Y—CO— des radicaux d'halogénures ou d'anhydrides d'acides réactifs, avec une di- ou une tri-hydroxyalkylamine $(HO)_{2\text{-}3}alkyl\text{-}NH_2$ dont les fonctions hydroxy peuvent être bloquées par acétalisation, et, dans le dérivé de bis-(hydroxyalkylamide) d'acide 5-(N-alkyl-α-hydroxyacyl-amino)-2,4,6-triiodo-isophtalique obtenu, on élimine la fonction bloquante A et on convertit les fonctions acétals éventuellement présentes en fonctions hydroxy par hydrolyse.

6. Agent de contraste aux rayons X caractérisé en ce qu'il contient en tant que composant opacifiant au moins un bis-(hydroxyalkylamide) d'acide 5-(N-alkyl-α-hydroxyacétyl-amino)-2,4,6-triiodo-isophtalique selon la revendication 1.